# EUROPEAN PATENT SPECIFICATION

(11) **EP 1 370 303 B1**
(45) Date of publication and mention of the grant of the patent: **17.08.2011**
(21) Application number: 02709900.1
(22) Date of filing: 31.01.2002
(51) Int. Cl.: A61L 2/26, A01N 63/00, A01N 41/02, A01N 41/04, A01N 41/10, A61L 2/00, A61L 2/08, A61L 2/16, A61L 2/025

(54) **PRION DISINFECTION**
PRION-DESINFEKTION
DESINFECTION PRIONIQUE

(30) Priority: 07.02.2001 AU PR293801
(43) Date of publication of application: 17.12.2003
(60) Divisional application: 10152838.8
(73) Proprietor: NOVAPHARM RESEARCH PTY. LTD., Rosebery, NSW 2018 (AU)
(72) Inventor: KRITZLER, Steven, Cronulla, NSW 2230 (AU); SAVA, Alex, Paddington, NSW 2021 (AU); ZALUNARDO, Michael, Koonawarra, NSW 2530 (AU)
(74) Representative: De Clercq, Ann G. Y.
(86) International application number: PCT/AU2002/000092
(87) International publication number: WO 2002/062400

(56) References cited:
- EP-A1- 0 358 500
- EP-A2- 0 742 018
- WO-A-02/053723
- WO-A1-99/42829
- WO-A1-99/51279
- RIESNER D. ET AL: 'Disruption of prion rods generates 10-nm spherical particles having high alpha-helical content and lacking scrapie infectivity' JOURNAL OF VIROLOGY vol. 70, no. 3, 1996, pages 1714 - 1722, XP002971239
- KOCISKO D.A. ET AL: 'Partial unfolding and refolding of scrapie-associated prion protein: evidence for a critical 16-kDa C-terminal domain' BIOCHEMISTRY vol. 35, 1996, pages 13434 - 13442, XP002150538
- BOLTON D.C. ET AL: 'Molecular characteristics of the major srapie prion protein' BIOCHEMISTRY vol. 23, 1984, pages 5898 - 5906, XP002961644
- DIENER T.O. ET AL: 'Viroids and prions' PROC. NATL. ACAD. SCI. USA vol. 79, 1982, pages 5220 - 5224, XP002971773

## Description

### TECHNICAL FIELD

This invention relates to compositions and methods for inactivating prions and to means for disinfecting materials contaminated by prions or by similar conformationally altered proteins.

### BACKGROUND ART

Historically, infectious agents such as bacteria, fungi, parasites, and viroids have well established methods of control that involve various forms of disinfection and sterilization (e.g. steam sterilization, dry sterilization, pasteurization, sterile filtration, treatment with ethylene oxide, glutaraldehyde, phenols or other disinfecting chemicals, radiation, etc.). With viruses, there are also established methods for example lowering the pH to 4.0 or below, heating at 60°C for extended periods, or use of organic solvents in high concentrations. In addition, UV treatment, formaldehyde and specific antiviral agents have been employed.

For some years now, new and previously unknown species of pathogenic agents have appeared and have been reported in scientific publications. These have been referred to as prions and present one of the greatest challenges facing the health care industry today. Prions are infectious particles that differ from bacteria and other previously known infectious agents. While there is no firm evidence on the exact structure of prions, a number of diseases have been identified recently both in humans and animals, that appear to be attributable to prions. As detailed in PCT/US00/14353 (the content of which is incorporated herein by reference), human diseases attributed to prions include Kuru, Creutzfeldt-Jakob disease (CJD), Gerstmann-Straussler-Scheinker disease (GSS), and Fatal Familial Insomnia.(FFI).

In addition to prion diseases of humans, disorders of animals are included in the group of known prion diseases. Scrapie of sheep and goats is perhaps the most studied animal prion disease. Several lines of inquiry have suggested a link between variant CJD and a preceding epidemic of bovine spongiform encephalopathy (BSE). No successful therapeutic treatments have been developed and as a result these diseases are always fatal. Adding to the problem is the fact that the incubation period can be up to 30 years in humans and this factor presents a major challenge to the scientists involved, with some predicting an epidemic "in the pipeline".

Groups possibly at risk of infection include patients who may come into contact with infected medical instruments during surgery, medical staff dissecting infected material, and healthcare workers responsible for cleaning and sterilizing instruments. There are also concerns that groups at risk may be broadened to include veterinarians, abattoir workers, butchers in contact with cows or beef primarily in Europe and more recently persons receiving blood transfusions or organs from donors incubating a prion disease.

The structure of prions has been the subject of intense investigation and different points of view have been expressed. Some scientists believe they are extremely small viruses, while most experts now believe that prions are actually infectious proteins without a DNA or RNA core. More particularly the consensus now is that the PrP gene of mammals expresses a protein which can be the soluble, non-disease, cellular form PrP^{c} or can be an insoluble disease form PrP^{sc}. Many lines of evidence indicate that prion diseases result from the transformation of the normal cellular form into the abnormal PrP^{Sc} form. There is no detectable difference in the amino acid sequence of the two forms. The PrP^{c} form is composed of a highly membrane associated 33 - 35kDa protein which degrades on digestion with protease K. However the PrP^{Sc} form has an altered conformational form, in particular having a high level of ß-sheet conformation. Properties of PrP^{Sc} useful in diagnosing the infective altered conformational form are a protease resistant core of 27 - 30kDa. Another distinctive feature of the altered conformational infective form is that it acquires a hydrophobic core.

Conventional disinfection and sterilizing agents have no significant effect on prions in an acceptable time. Attempts to deactivate prions and/or to disinfect surfaces on which they may be transmitted have shown an extraordinary resistance. The conditions required are generally too severe to be practical for routine disinfection, not only in terms of time and cost, but also in terms of damage to materials and occupational health hazards involved. For example in one study infectious PrP^{Sc} particles have been detected in a sample after 5-15mins /600°C dry heat although total destruction could be achieved at 1000°C in 15mins and in from 1-10hrs at >200°C. It has been proposed to treat with I M. caustic soda (pH14) for 2hrs but that treatment is extremely corrosive, dangerous to staff, and aggressive to materials. US5633349 describes a procedure for treating a biological material involving treatment with 6-8 molar urea or 1-2 molar sodium thiocyanate for a minimum of 12 hrs (preferably 18 hrs) which suffers from similar disadvantages.

Because of the difficulties in decontamination it has been proposed as preferably that surgical instruments used in brain surgery should be used only once, but this implies a disposal risk in addition to being expensive and for some instruments impractical. PCT/US00/14353 describes a method of rendering prions non-infectious by use of a polycationic dendrimer but it is not clear whether that process is reversible or permanent or commercially viable for disinfecting surfaces.

Although attention has been focused on the PrP^{c} form and the PrP^{Sc} form it has also been suggested that the protein can exist in an intermediate form which has a ß-sheet content intermediate between the predominantly alpha helix structure of the PrP^{c} form and the predominantly ß-sheet conformation of the PrP^{Sc} form and which retains solubility in the absence of a denaturant.

The assembly or misassembly of normally soluble proteins into conformationally altered insoluble proteins is thought to be causative of, or implicated in, a variety of other diseases. Although the invention will be herein described in relation to prions, it will be understood to be applicable to other insoluble or enzyme resistant conformationally altered proteins implicated in disease.

The above discussion of prior art is not to be construed as an admission with regard to the common general knowledge in Australia.

An object of the invention is to provide improved, or at least alternative, means of disinfecting a surface infected with prions. In certain preferred embodiments, the invention renders prions inactive more efficiently, that is to say more effectively in a given time, or as effectively in a shorter time, than prior art methods. Certain highly preferred embodiments of the invention achieve better than a 4 log reduction in less than 60 mins at below 60°C. In some embodiments the invention is also applicable to prions in situations other than on surfaces for example in suspension in a solid, liquid or gaseous medium or in biological systems and may have other in vitro or in vivo uses. It is an object of some disclosures of the application provide improved diagnostic tools and of other embodiments to provide novel epimers for preparation of antibodies.

The term "prion protein" as herein used includes variants, fragments, fusions, and analogues that have other interactions or activities that are substantially the same as those of a full length prion protein sequence, but which may be more convenient to use and includes all forms of secondary structure The term is also herein used to include prion surrogates, that is to say proteins which are not themselves prions but which have similar structure or exhibit similar behaviour to prions and can be used to model or predict how a prion would perform under specified conditions. The term "PrP^{Sc} prion protein" is intended to have a similarly broad meaning but is limited to prion proteins which by virtue of their secondary or tertiary structure are enzyme resistant and includes conformations which are similarly enzyme resistant

### DESCRIPTION OF THE INVENTION

According to a first aspect the present application provides a method of disinfection comprising the steps of treating a surface contaminated with a PrP^{Sc} prion protein or a surrogate thereof simultaneously with a combination of (1) one or more enzymes effective to cleave a prion protein to fragments having a non-infective molecular weight, and (2) one or more agents selected to favour conformational unfolding of the PrP^{Sc} prion protein while not denaturing the one or more enzymes.

According to a second aspect the application provides a method according to the first aspect, further including (3) one or more agents selected to promote or protect folding of the one or more enzymes, without preventing cleavage of the prion protein. Preferably, the conditions are selected to favour unfolding over refolding.

It is presently accepted that proteins having a molecular weight of less than 27kDa are noninfective and safe, and accordingly the method of the application envisages digestion or cleavage of the prion to fragments of which at least 90% and preferably at least 98% are less than 27kDa, and preferably less than 25kDa or more preferably less than 23kDa. However, if in the future a protein of less than 27 kDa should be found to be infective, the method of the application could be utilized to fragment the protein to fragments of any safe size.

The term "prion surrogate" as used herein is according to the FDA definition, that is to say, proteins having a similar resistance to proteases due to the presence of β-folding.

The term "agent" is herein used to include both chemical reagents for example anionic surfactants, reagents to modify pH, and also non-chemical agents which effect physical and/or thermodynamic conditions such as pressure, temperature, irradiation and other energetic influences which promote folding, or unfolding, as the context requires. Folding agents are sometimes referred to as "refolding" agents. Unfolding agents are sometimes referred to as "denaturing" agents

According to a third aspect the disclosure provides a method according to the first or second aspect wherein said one or more agents selected to favour conformational unfolding includes one or more agents selected from the group consisting of irradiation, electric field, magnetic field, energetic vibration and combinations thereof.

In other disclosures a combination of chemical and physical agents is employed, for example the agents of step (2) include an anionic surfactant in combination with sonication by ultrasound.

For preference the prion is subjected to sound waves in the ultrasonic range during the treatment. However the unfolding may be induced or aided by other forms of radiation such as microwave radiation, radiation in the radiofrequency, infra red, visible or U.V spectrum, sound at audible or lower frequency, energetic vibration from mechanical means such as magnetic or vortex stirring. Other forms of energetic input may include from electron beam irradiation, laser irradiation, or electrolysis.

According to other aspects, the disclosures include compositions for use in conducting the method, to novel prion fragments produced by the method and to novel antibodies produced from said fragments

According to the disclosures a contaminated surface, for example a surgical instrument contaminated with a PrP^{Sc} protein, is treated with a combination of (1) one or more enzymes effective to cleave the prion protein into fragments of a non infective molecular weight, (currently, less than 27kDa), and (2) one or more agents selected to favour conformational unfolding of the prion protein

PrP^{Sc} protein is characteristically resistant to attack by enzymes including proteolytic enzymes. Without wishing to be bound by theory, the present inventors supposed that the resistance of PrP^{Sc} protein to attack by enzymes is a consequence of the folded conformation (having a high ratio of ß-sheet secondary structure relative to alpha helix structure). The disclosure involves the conception that it is possible to select one or more agents so as to promote, under selected conditions, unfolding of the PrP^{Sc} protein sufficiently for an enzyme to gain access and cleave PrP^{Sc} protein.

Many proteins are prone to loose their natural three dimensional folding pattern ("secondary and tertiary structure") and to become "denatured". The denaturation includes breakdown of the intramolecular interaction, especially hydrogen and disulphide bonds, and thus the loss of the secondary structure which virtually all native proteins have in at least parts of the molecule, and which generally is decisively responsible for the activity of the protein.

Those skilled in the art appreciate that enzymes are themselves proteins and tend to be readily denatured by agents which promote protein unfolding.

It is not clear whether that is because the unfolding agent binds to the enzyme, preventing the enzyme from binding to a target substrate, or more likely because the unfolding agent promotes unfolding of the enzymes conformational structure, rendering it inactive or "denatured" or a combination of those effects. PrP^{Sc} protein on the other hand is highly resistant to unfolding. It has hitherto been considered impossible to formulate a system in which an enzyme retains activity in the presence of an unfolding agent effective to influence such an intractable protein as PrP^{Sc}. Surprisingly the present inventors have found that either (i) certain unfolding agents selectively unfold or relax the PrP^{Sc} protein while not unfolding (denaturing) a selected enzyme or (ii) that folding and unfolding agents can be combined in such a way that the folding agent selectively promotes or retains enzyme activity, while the unfolding agent selectively and sufficiently unfolds the prion to provide access to the enzyme for prion scission.

These intrinsically conflicting desiderata are met in the present application by classifying agents as promoting "folding" or "unfolding" and then determining their relative effect on enzymes and on PrP^{Sc} protein or a surrogate thereof.

The surface may be first treated with the one or more agents and the one or more enzymes may be added subsequently but in preferred embodiments the surface undergoing treatment is subjected to both simultaneously.

The disclosed enzyme may be a proteolytic enzyme. Such enzymes are:
- non-specific proteinases:- e.g. serine-, aspartic-, metalloproteinases
- more specific proteinases -e.g. keratinases, collagenases etc
- any other enzyme(s) that posses proteolytic activity

The one or more agents selected to favour conformational unfolding of the prion protein are chosen to be effective to provider access by the enzyme to the prion protein.

In general the folding-unfolding of a polypeptide chain may be a thermodynamically reversible equilibrium process or may be irreversible.

By way of example only, agents which promote unfolding (denaturation) include: -
(1) heat - increases in temperature up to about 150° C.
(2) pH - values below 3 and above 9 (global effect resulting from ionization of many side chain residues accessible to the solvent) or in some molecules may be attributable to local effects due to ionization of specific groups (e.g. serine proteases due ionization of N-terminal amino group of the carboxylate)
(3) Selected organic solvents of a kind which tend to denature, dissolve or swell proteins. Generally the products are not completely unfolded and possess an ordered conformation which differs from the native state. Solvents which favour helical conformations (i.e. unfolding) are exemplified by N-dimethylformamide, formamide, m-cresol, dioxan, CHCl₃, pyridine, dichlorethylene, and 2-chloroethanol. This group also includes solvents which have a weak tendency to form hydrogen bonds such as the alcohols, ethanol, n-propanol, methanol (especially in mixture with 0.01%HCl), Also, solvents which tend to disorganize the structure e.g. dimethylsulphoxide (DMSO) at high concentrations, dichloroacetic acid and trifluoroacetic acid, and other electrophillic solvents
(4) Certain organic solutes and chaotropic agents. - Such as urea, guanidine hydrochloride (GuHCl). The transition to randomly coiled polypeptide is complete for 6-8M GuHCl at room temp except for some exceptionally stable proteins. These agents may be markedly influenced by temperature, pH other reagents and conditions.
(5) Certain surfactants - Ionic surfactants tend to bind to proteins and initiate unfolding of tertiary structure. Anionic detergents are very strong denaturants. E.g. Sodium dodecyl sulfate (SDS) is able to completely unfold many (but not all) proteins at concentrations close to the critical micelle concentration. Dodecyl benzene sulfonate is also a denaturant. The detergents do not necessarily result in a complete unfolding since in some cases it appears that the hydrophobic part of the detergent might interact with the ordered structure of the protein to form micellar regions. Cationic surfactants are usually less effective unfolding agents than anionic. Dodecyl ethoxy sulfates tendency to denature bovine serum albumin (BSA) decreases with increasing ethoxy groups and disappears for ethoxy greater than six
(6) Inorganic salts can induce conformational transitions in proteins. For example LiBr, CaCl₂, KSCN, NaI, NaBr, sodium azide are strong denaturants. Although these salts do not necessarily lead to completely unfolded protein, the residual ordered structure may be disrupted by energy input e.g. increasing temperature. Anions such as CNS⁻> Γ > Br⁻ > NO₃⁻> Cl⁻ >CH₃COO⁻ > SO₄⁻ exhibit similar behaviour as do guanidinium salts and tetraalkyl ammonium salts However (GuH)₂SO₄ has been observed to protect certain proteins against denaturation.
(7) agents which cause scission of the S - S bond such as thioglycols
(8) Other substances with strong affinity to either hydrophilic or hydrophobic residues of amino acids
(9) Adsorption on certain surfaces and interfaces including zeolites, including air/liquid interfaces.
(10) Ultrasonic energy, Infra red and microwave radiation, high pressure, and subjecting protons to the action of electric and/or magnetic fields may be able to promote unfolding (refolding), and even shaking or stirring may be influential.

In forms of the application a combination of agents is used for example a surfactant and /or a suitable solvent with ultrasound is employed. It is unclear whether the input of energy, such as from ultrasound, assists in driving the folding/unfolding equilibrium in favour of unfolding of the PrP^{Sc} protein at a greater rate than it does in denaturing the enzyme, or whether it merely assists in providing access of the reagents or enzymes to the prion, or whether it is effective in activating the enzyme. Other methods of applying energy include application of sound waves in the sub-sonic range. However energetic vibration may be induced by other forms of electromechanical radiation or energetic vibration from mechanical means such as magnetic or vortex stirring. Other forms of energetic input may include from electron beam irradiation, laser, or electrolysis.

As indicated above, most of the discussed unfolding agents would be expected effectively to denature the enzyme. Either the unfolding agent and its conditions of use must be carefully selected so as to permit digestion of the PrP^{Sc} protein or its surrogate without denaturing the enzyme, or alternatively the unfolding agent must be combined with a folding agent.

Folding agents as disclosed include:
(1) Nucleophillic solvents and highly hydrogen bonded organic solvents. There is competition between the energy of the peptide hydrogen bonds and the strength of hydrogen bonds between solvent molecules. When solvent molecules are linked by strong hydrogen bonds the equilibrium is shifted to towards stabilization of peptide hydrogen bonds. Solvents such as dioxan, acetonitrile, dimethylformamide, pyridine, and, at low concentrations, dimethylsulphoxide(DMSO) which are good proton acceptors but weak proton donors have a very weak tendency to disrupt peptide hydrogen bonds and tend to induce ordered conformation, especially in globular proteins.
(2) Stabilizing solvents such as polyhydric alcohols (e.g. glycerol, ethylene glycol, and propylene glycol, sucrose and the like) which are weakly protic and in the presence of which proteins tend to remain conformationally stable and may be used as stabilizing agents.
(3) Non-ionic surfactants such as alkyl, phenyl or alkyl ethoxylates, propoxylates or copolymers thereof, alkylpolyglucosides etc, sarcosinates (e.g. sodium- (N-lauroyl) sarcosinate) do not alter the tertiary structure of protein and any unfolding occurs in the region of the isotherm where a significant increase in surfactant binding by non-specific cooperative interaction begins The effects of SDS can be reduced by addition of non-ionic or amphoteric surfactants
(4) Zwitterionic and amphoteric surfactants,
(5) High concentrations of buffers, (e.g. phosphates, acetates, citrates, borates)
(6) Surface active homo-, co-, or block- polymers which contain weakly hydrophobic and weakly hydrophilic zones in alternating arrangement,
(7) Protective agents such as sulphated compounds, deoxycholate, glycosaminoglycans If a folding agent is combined with an unfolding (denaturing) agent then the agents and conditions must be selected to protect or refold the enzyme while irreversibly unfolding or at least opening the prion sufficiently for access by the enzyme. For example, the pH and/or temperature may be selected so that the folding agent acts selectively on the enzyme while the unfolding agent acts selectively on the PrP^{Sc} protein.

### BEST MODES FOR CARRYING OUT THE INVENTION

Bovine albumin with high globulin content (Sigma product A7906), beta-galactosidase (G7279) and rabbit muscle myosin (M0163)) were used as models of proteins with low-solubility and high beta-sheet content. The molecular weight of the above proteins are significantly larger than that of the prions. The identifying of molecular mass of peptide fragments after enzymatic digest is easier, as most of proteases used in the experiment have molecular mass (20-35 KDa) similar to prions.

SDS-PAGE was conducted using the method described by Laemmli U.K. , Nature, 227, pages 680-685,(1970). The protein solution was boiled for 2 min in sample buffer containing 2% SDS. 1.5mm polyacrylamide slab gels (8-12%) were loaded with 10 microlitres of the protein per lane, and subjected to non-reducing conditions (ie. no betamercaptoethanol in sample buffer). Electrophoresis was performed for 1 hour at 150 V, until the dye front was at the bottom of the gel. The gel was then removed and the protein bands visualized by staining either with Coomassie brilliant blue R-250 (Sigma) or silver stained (Bio-Rad). The molecular mass value for proteins was determined by using calibrating curve obtained with prestained low molecular weight markers (Bio-Rad marker 161-0318).

Cleaving the proteins was considered sufficient when no fragments with molecular mass larger than 14.4 KDa (the molecular weight of lysozyme) were detected after the combined action of unfolding agents and enzymes. That indicates that the treatment was effective on the surrogate.

When peptide fragments with molecular mass of larger than 14.4 KDa were present, the results was reported as positive

To prove that the method of the invention may be used to deactivate prions, and not merely cleave the surrogate, the prion detection test developed by Prionics AG was employed. The "Prionics-Check" is an immunological test for the detection of prions in animal tissues that use a novel antibody developed by PRIONICS AG. The PrP^{Sc} remaining in the reaction mixture is bound by the antibody and detected using an enzyme coupled to the antibody.

100 mL aliquots of the solutions as described in table 1 were spiked with approx. 1 microgram of recombinant prion protein and the Prionics Check was performed as per the procedure described in Appendix 1.

When PrP^{Sc} was detected after the enzymatic digest, the results were reported as positive Table 1 shows that in control experiments 1-1 to 6-1 fragments having a mass greater than 14.4 KDa were detected as was PrP^{Sc}. However in experiments 1-2 to 6-2 no fragments were detected with a mass greater than 14.4 KDa and no PrP^{Sc} was detected. 1-1 differs from 1-2 by inclusion of an unfolding agent (3% DOBS) shown to be effective in 30 min at 70° C

2-2 and 3-2 differ from 2-1 and 3-1 respectively by inclusion of sonication. In 2-2 an unfolding agent 3% DOBS in combination with 25%Terric 164 ( a folding agent) was effective at 25° C with sonication at 40kHz In 3-2 a similar result was obtained with 10%SDS as unfolding agent and a zwitterionic surfactant as folding agent at 2.6 mHz 4-2 differs from 4-1 by the combination of borax with SDS at a more elevated temperature. 5-2 differs from 5-1 by combining DOBS with Triton X-100 in the absence of boron 6-2 differs from 6-1 by increasing the concentration of DMSO from reversibly unfolding (0.05%) to irreversibly unfolding (0.5%)

**TABLE 1**

| No. | Test procedure/solutions | SDS-PAGE Albumin | SDS-PAGE beta-galacto sidase | SDS-PAGE myosin | Prionics Check |
|---|---|---|---|---|---|
| 1-1. | Distilled water | + | + | + | + |
| | Warm to 70C | | | | |
| | **Keep in water bath at 70C for 30** min, 15 units protease activity per mL, pH 9 | | | | |
| | Cool down to 25C | | | | |
| 1-2. | 3% DOBS and Distilled water *diluted 1:100* | - | - | - | - |
| | Warm to 70C | | | | |
| | **Keep in water bath at 70C for 30** min, 15 units protease activity per mL, pH 9 | | | | |
| | Cool down to 25C | | | | |
| 2-1. | 3% DOBS, 25% Teric 164, *diluted 1:100* | + | + | + | + |
| | 15 units protease activity per mL pH 9 | | | | |
| | **25C for 30 min** | | | | |
| 2-2 | 3% DOBS, 25% Teric 164 *diluted 1:100* 15 units protease activity per mL | - | - | - | - |
| | **sonicated with 40 kHz ultrasound 25C for 30 min** | | | | |
| 3-1 | 10% SDS 10% Empigen BS/AU(zwitterionic surfactant) *diluted 1:100* | + | + | + | + |
| | 15 units protease activity per mL pH 9 | | | | |
| | **25C for 30 min** | | | | |
| 3-2 | 10% SDS 10% Empigen BS/AU(zwitterionic surfactant) *diluted 1:100* | - | - | - | - |
| | 15 units protease activity per mL pH 9 | | | | |
| | **25C sonicated 2.6mHz for 30 min** | | | | |
| 4-1 | 10% SDS, 4% borax *diluted 1:100* | + | + | + | + |
| | 15 units protease activity per mL pH 9 | | | | |
| | **25C for 30 min** | | | | |
| 4-2 | 10% SDS, 4% borax *diluted 1:100* | - | - | - | - |
| | 15 units protease activity per mL pH 9 | | | | |
| | **55C for 30 min** | | | | |
| 5-1 | 15% DOBS, 5% Triton X100 4% Borax *diluted 1:100* | + | + | + | + |
| | 15 units protease activity per mL pH 9 | | | | |
| | **25C for 30 min** | | | | |
| 5-2 | 15% DOBS, 5% Triton X100 *diluted 1:100* | - | - | - | - |
| | 15 units protease activity per mL pH 9 | | | | |
| | **25C for 30 min** | | | | |
| 6-1 | *.05% DMSO* | + | + | + | + |
| | 15 units protease activity per mL pH 9 | | | | |
| | **25C for 30 min** | | | | |
| 6-2 | .5% *DMSO* | - | - | - | - |
| | 15 units protease activity per mL pH 9 | | | | |
| | **25C for 30 min** | | | | |

| | | | | | |
|---|---|---|---|---|---|
| DOBS = dodecyl benzene sulfonic acid (Sigma Product No. D2525) DMSO =dimethylsulfoxide (Sigma Product No. D5879) Protease = Subtilisin Carlsberg (Sigma Product No. P5380) Sonication at 40 kHz performed using ultrasonic bath supplied by UNISONICS Pty Ltd. Sonication at 2.6mHz performed using Disonics Pty Ltd. ultrasonic nebulizer. | | | | | |

### APPENDIX 1

### PRIONICS CHECK TEST METHOD

The protocol below outlines using the protease resistant core of PrP^{sc}, from a recombinant source known to be representative of the naturally occurring infectious agent. It has been proven experimentally that the protease resistant core of the prion is not infectious, but indicates the presence of infectious agents
1. Weigh 1 microgram of PrP^{sc} or BSE infected animal brain homogenate that contains 1 mcg or PrP^{Sc} and reconstitute it in 1 ml of deionised water
2. Add to 10ml of test solution and subject to appropriate deactivation protocol
3. Take 10 microlitre aliquot of the test solution and add it to 10 microlitres of sample buffer
4. Perform SDS-PAGE of
   - untreated PrP^{sc} solution used for spiking (positive control)
   - solution under study

All proteins or protein fragments are separated in an electric field according to their size. The small proteins migrate faster than the large proteins. After a period of time the smallest fragments of the decomposed prion proteins migrate out of the gel while the resistant PrP^{Sc} fragments will be present in the lower half of the gel. In the control sample where the prion protein remains resistant to protease, non-cleaved PrP^{Sc} molecules will remain higher up in the gel.
1. Transfer proteins from the gel to nitrocellulose membrane by Western blotting.
2. Add monoclonal antibodies (Prionics Product No. 01-020).
3. Allow to bind to the proteins and then wash away non-bound antibodies.
4. The horseradish-peroxidase conjugated to primary antibodies is allowed to react with a chemoluminescence substrate (ECL product No. RPN 2209 supplied by AMERSHAM Life Science)
5. Expose the membrane to X-Ray film, develop the film.
6. 10. Assess whether prion protein are present. Report results as positive when the antibodies are retained at the position corresponding to molecular mass of prion protein.

## Claims

1. A non-therapeutic method of disinfection comprising the steps of treating a surface, suspension or solution contaminated with a PrP^{Sc} prion protein or a surrogate thereof simultaneously with a combination of
(1) subtilisin carlsberg, and
(2) either :
a) dodecyl benzene sulfonic acid (DOBS) Teric 164 and sonication;
b) sodium dodecyl sulfate (SDS), Empigen BS/AU and sonication;
c) SDS, borax and a temperature increased up to 150°C;
d) DOBS and Triton X100; or
e) dimethylsulfoxide (DMSO) at an irreversibly unfolding concentration,
wherein DOBS, SDS, DMSO, temperature and sonication favour conformational unfolding of the PrP^{Sc} prion protein while not denaturing the subtilisin Carlsberg and Teric 164,Empigen BS/AU , borax and Triton-X 100 promote or protect folding of the , without preventing the cleavage of the prion protein.

2. A method according to claim 1, wherein the treatment is selected so as to result after cleavage in a predetermined percentage of the protein fragments having a molecular weight of less than a predetermined molecular weight.

3. A method according to claim 1 or claim 2, wherein at least 90% of the protein fragments after cleavage have a molecular weight of less than 27kDa.

4. A method according to claim 1 or claim 2, wherein at least 90% of the protein fragments after cleavage have a molecular weight of less than 25kDa.

5. A method according to claim 1 or claim 2, wherein at least 90% of the protein fragments after cleavage have a molecular weight of less than 23kDa.

6. A method according to any of claims 1 to 5, wherein the conditions are selected to protect or refold the subtilisin Carlsberg while irreversibly unfolding or at least opening the prion sufficiently for access by the subtilisin Carlsberg

7. A method according to any one of the preceding claims, further including in the combination one or more agents selected to favour conformational unfolding selected from the group consisting of irradiation, electric field, magnetic field, energetic vibration and combinations thereof.

8. A method according to claim 7, wherein the energetic vibration is one or more of ultrasound, electromagnetic or mechanical vibration.

9. A method according to any one of the preceding claims, further including in the combination one or more agent selected to promote unfolding selected from the group consisting of heat, pH, organic solvents of the kind which tend to denature proteins, chaotropic agents, surfactants tending to bind proteins, inorganic salts which are strong denaturants of proteins, agents which cause S-S bond scission, substances having a strong affinity for hydrophilic residues of amino acids, substances having a strong affinity for hydrophobic residues of amino acids, substances promoting adsorption on surfaces and combinations of the foregoing.

10. A method according to any one of the preceding claims, further including in the combination one or more folding agents selected from the group consisting of nucleophilic solvents, weakly protic stabilizing solvents, non ionic surfactants, ionic surfactants, zwitterionic and amphoteric surfactants, buffers, surface active homo, coor block-co-polymers, sulphated compounds, deoxycholate, glycosaminoglycans and combinations of the foregoing.

11. A composition for treating a surface contaminated with a PrP^{Sc} prion protein or a surrogate thereof comprising
(1) subtilisin car/sberg
, and
(2) either :
a) DOBS and Teric 164;
b) SDS and Empigen BS/AU
c) SDS and borax;
d) DOBS and Triton X100; or
e) DMSO at an irreversibly unfolding concentration,
wherein DOBS, SDS and DMSO flavour conformational unfolding of the PrP^{Sc} prion protein while not denaturing the subtilisin Carlsberg and Teric 164, Empigen BS/AU borax and Triton-X 100 promote or protect folding of the subtilisin Carlsberg, without preventing the cleavage of the prion protein.

12. A composition according to claim 11, wherein the agent is selected so as to result after cleavage in a predetermined percentage of the protein fragments having a molecular weight of less than a predetermined molecular weight.

13. A composition according to claim 11, wherein the agent is selected so as to result after cleavage in at least 90% of the protein fragments after cleavage having a molecular weight of less than 27kDa.

14. A composition according to claim 11, wherein the agent is selected so as to result after cleavage in at least 90% of the protein fragments having a molecular weight of less than 25kDa.

15. A composition according to claim 11, wherein the agent is selected so as to result after cleavage in at least 90% of the protein fragments having a molecular weight of less than 23kDa.

## Patentansprüche

1. Nichttherapeutisches Verfahren zur Desinfektion, welches die folgenden Schritte umfasst: gleichzeitiges Behandeln einer Oberfläche, einer Suspension oder einer Lösung, die mit einem PrP^{Sc}-Prionenprotein oder einem Surrogat desselben verunreinigt ist, mit einer Kombination aus:
(1) Subtilisin Carlsberg, und
(2) entweder:
a) Dodecylbenzolsulfonsäure (DOBS), Teric 164 und Beschallung;
b) Natriumdodecylsulfat (SDS), Empigen BS/AU und Beschallung;
c) SDS, Borax und eine Erwärmung auf bis zu 150°C;
d) DOBS und Triton X 100 ; oder
e) Dimethylsulfoxid (DMSO) in einer Konzentration für irreversible Entfaltung,
wobei durch DOBS, SDS, DMSO, Temperatur und Beschallung eine konformere Entfaltung des PrP^{Sc}-Prionenproteins unterstützt wird und dabei das Subtilisin Carlsberg nicht denaturiert wird, und Teric 164, Empigen BS/AU Borax und Triton-X 100 die Faltung des Subtilisin Carlsberg unterstützen oder schützen, ohne die Spaltung des Prionenproteins zu verhindern.

2. Verfahren nach Anspruch 1, wobei die Behandlung so gewählt wird, dass sie nach der Spaltung zu einem vorgegebenen Prozentsatz der Proteinfragmente führt, welche eine Molekularmasse von weniger als einer vorgegebenen Molekularmasse aufweisen.

3. Verfahren nach Anspruch 1 oder Anspruch 2, wobei mindestens 90 % der Proteinfragmente nach der Spaltung eine Molekularmasse von weniger als 27 kDa aufweisen.

4. Verfahren nach Anspruch 1 oder Anspruch 2, wobei mindestens 90 % der Proteinfragmente nach der Spaltung eine Molekularmasse von weniger als 25 kDa aufweisen.

5. Verfahren nach Anspruch 1 oder Anspruch 2, wobei mindestens 90 % der Proteinfragmente nach der Spaltung eine Molekularmasse von weniger als 23 kDa aufweisen.

6. Verfahren nach einem der Ansprüche 1 bis 5, wobei die Bedingungen so gewählt werden, dass das Subtilisin Carlsberg geschützt wird oder sich neu faltet, während das Prion für einen Zugriff durch das Subtilisin Carlsberg in ausreichendem Maße irreversibel entfaltet oder zumindest geöffnet wird.

7. Verfahren nach einem der vorhergehenden Ansprüche, wobei die Kombination ein oder mehrere Mittel umfasst, die so gewählt werden, dass eine konformere Entfaltung unterstützt wird, und aus der Gruppe ausgewählt werden, die aus Bestrahlung, einem elektrischen Feld, einem Magnetfeld, energiereichen Schwingungen und Kombinationen daraus besteht.

8. Verfahren nach Anspruch 7, wobei es sich bei den energiereichen Schwingungen um eines oder mehreres aus Ultraschall, elektromagnetischen Schwingungen und mechanischen Schwingungen handelt.

9. Verfahren nach einem der vorhergehenden Ansprüche, wobei die Kombination ferner ein oder mehrere Mittel umfasst, die so gewählt werden, dass eine Entfaltung unterstützt wird, und aus der Gruppe ausgewählt werden, die aus Wärme, Einstellen des pH-Werts, organischen Lösungsmitteln der Art, welche gewöhnlich Proteine denaturieren, chaotropen Mitteln, oberflächenaktiven Substanzen, welche gewöhnlich Proteine binden, anorganischen Salzen, welche Denaturierungsmittel für Proteine sind, Mitteln, welche die Spaltung von S-S-Bindungen bewirken, Substanzen, welche eine starke Affinität zu hydrophilen Aminosäureresten besitzen, Substanzen, welche eine starke Affinität zu hydrophoben Aminosäureresten besitzen, Substanzen, welche die Adsorption auf Oberflächen unterstützen, und Kombinationen der vorstehenden Mittel besteht.

10. Verfahren nach einem der vorhergehenden Ansprüche, wobei die Kombination ferner ein oder mehrere Faltungsmittel umfasst, die aus der Gruppe ausgewählt werden, die aus nukleophilen Lösungsmitteln, schwach protischen stabilisierenden Lösungsmitteln, nichtionischen oberflächenaktiven Substanzen, ionischen oberflächenaktiven Substanzen, zwitterionischen und amphoteren oberflächenaktiven Substanzen, Puffern, oberflächenaktiven Homopolymeren, Copolymeren oder Blockcopolymeren, sulfatierten Verbindungen, Desoxycholat, Glykosaminoglykanen und Kombinationen der vorstehenden Mittel besteht.

11. Zusammensetzung zum Behandeln einer Oberfläche, die mit einem PrP^{Sc}-Prionenprotein oder einem Surrogat desselben verunreinigt ist, wobei die Zusammensetzung das Folgende umfasst:
(1) Subtilisin Carlsberg, und
(2) entweder:
a) DOBS und Teric 164;
b) SDS und Empigen BS/AU;
c) SDS und Borax;
d) DOBS und Triton X 100 oder
e) DMSO in einer Konzentration für irreversible Entfaltung,
wobei durch DOBS, SDS, DMSO, Temperatur und Beschallung eine konformere Entfaltung des PrP^{Sc}-Prionenproteins unterstützt wird und dabei das Subtilisin Carlsberg nicht denaturiert wird, und Teric 164, Empigen BS/AU Borax und Triton-X 100 die Faltung des Subtilisin Carlsberg unterstützen oder schützen, ohne die Spaltung des Prionenproteins zu verhindern.

12. Zusammensetzung nach Anspruch 11, wobei das Mittel so ausgewählt ist, dass es nach der Spaltung zu einem vorgegebenen Prozentsatz der Proteinfragmente führt, welche eine Molekularmasse von weniger als einer vorgegebenen Molekularmasse aufweisen.

13. Zusammensetzung nach Anspruch 11, wobei das Mittel so ausgewählt ist, dass es dazu führt, dass nach der Spaltung mindestens 90 % der Proteinfragmente eine Molekularmasse von weniger als 27 kDa aufweisen.

14. Zusammensetzung nach Anspruch 11, wobei das Mittel so ausgewählt ist, dass es dazu führt, dass nach der Spaltung mindestens 90 % der Proteinfragmente eine Molekularmasse von weniger als 25 kDa aufweisen.

15. Zusammensetzung nach Anspruch 11, wobei das Mittel so ausgewählt ist, dass es dazu führt, dass nach der Spaltung mindestens 90 % der Proteinfragmente eine Molekularmasse von weniger als 23 kDa aufweisen.

## Revendications

1. Méthode non thérapeutique de désinfection comprenant les étapes de traitement d'une surface, d'une suspension ou d'une solution contaminée avec une protéine prion PrP^{Sc} ou un substitut de celle-ci, par combinaison simultanée de:
(1) subtilisin Carlsberg, et
(2) soit :
a) acide dodécylbenzène sulfonique (DOBS), Teric 164 et sonication;
b) sodium dodécyl sulfate (SDS), Empigen BS/AU et sonication;
c) SDS, borax et une augmentation de température jusqu'à 150°C;
d) DOBS et Triton X100 ; ou
e) diméthyl sulfoxide (DMSO) à une concentration à laquelle le dépliement est irréversible,
dans laquelle DOBS, SDS, DMSO, température et sonication favorisent le dépliement conformationnel de la protéine prion PrP^{Sc} tout en ne dénaturant pas la subtilisin Carlsberg, et Teric 164, Empigen BS/AU, borax et Triton-X 100 promeuvent ou protègent le repliement de la subtilisin Carlsberg, sans empêcher le clivage de la protéine prion.

2. Méthode selon la revendication 1, dans laquelle le traitement est sélectionné de manière à résulter après clivage en un pourcentage prédéterminé de fragments de protéine ayant un poids moléculaire inférieur à un poids moléculaire prédéterminé.

3. Méthode selon la revendication 1 ou la revendication 2, dans laquelle au moins 90% des fragments de protéine après clivage ont un poids moléculaire inférieur à 27 kDa.

4. Méthode selon la revendication 1 ou la revendication 2, dans laquelle au moins 90 % des fragments de protéine après clivage ont un poids moléculaire inférieur à 25 kDa.

5. Méthode selon la revendication 1 ou la revendication 2, dans laquelle au moins 90 % des fragments de protéine après clivage ont un poids moléculaire inférieur à 23 kDa.

6. Méthode selon l'une quelconque des revendications 1 à 5, dans laquelle les conditions sont sélectionnées pour protéger ou replier la subtilisin Carlsberg tout en dépliant de manière irréversible ou du moins tout en ouvrant le prion de manière suffisante pour le rendre accessible à la subtilisin Carlsberg.

7. Méthode selon l'une quelconque des revendications précédentes, incluant en outre dans la combinaison un ou plusieurs agents sélectionnés pour favoriser le dépliement conformationnel, sélectionnés parmi le groupe constitué de: irradiation, champ électrique, champ magnétique, vibration énergétique et combinaisons de ceux-ci.

8. Méthode selon la revendication 7, dans laquelle la vibration énergétique est l'une ou plusieurs parmi les ultrasons, vibration électromagnétique ou mécanique.

9. Méthode selon l'une quelconque des revendications précédentes, incluant en outre dans la combinaison un ou plusieurs agents sélectionnés pour promouvoir le dépliement, sélectionnés parmi le groupe constitué de chaleur, pH, solvants organiques du type de ceux qui ont tendance à dénaturer les protéines, agents chaotropiques, tensioactifs ayant tendance à lier les protéines, sels inorganiques qui sont de forts dénaturant des protéines, agents qui provoquent la scission des liaisons S-S, substances ayant une forte affinité pour les résidus hydrophiles des acides aminés, substances ayant une forte affinité pour les résidus hydrophobes des acides aminés, substances promouvant l'adsorption sur des surfaces et les combinaisons de ce qui précède.

10. Méthode selon l'une quelconque des revendications précédentes, incluant en outre dans la combinaison un ou plusieurs agents de repliement sélectionnés parmi le groupe constitué des solvants nucléophiles, solvants stabilisants faiblement protiques, tensioactifs non ioniques, tensioactifs ioniques, tensioactifs zwitterioniques et amphotères, tampons, tensioactifs homopolymères, copolymères ou copolymères à blocs, composés sulfatés, déoxycholate, glycosaminoglycanes et combinaisons de ce qui précède.

11. Composition pour le traitement d'une surface contaminée avec une protéine prion PrP^{Sc} ou un substitut de celle-ci comprenant :
(1) subtilisin Carlsberg, et
(2) soit :
a) DOBS et Teric 164 ;
b) SDS et Empigen BS/AU ;
c) SDS et borax ;
d) DOBS et Triton X100 ; ou
e) DMSO à une concentration à laquelle le dépliement est irréversible,
dans laquelle DOBS, SDS et DMSO favorisent le dépliement conformationnel de la protéine prion PrP^{Sc} tout en ne dénaturant pas la subtilisin Carlsberg, et Teric 164, Empigen BS/AU, borax et Triton-X 100 promeuvent ou protègent le repliement de la subtilisin Carlsberg, sans empêcher le clivage de la protéine prion.

12. Composition selon la revendication 11, dans laquelle l'agent est sélectionné de manière à résulter après clivage en un pourcentage prédéterminé de fragments de protéine ayant un poids moléculaire inférieur à un poids moléculaire prédéterminé.

13. Composition selon la revendication 11, dans laquelle l'agent est sélectionné de manière à résulter après clivage en ce qu'au moins 90 % des fragments de protéine après clivage ont un poids moléculaire inférieur à 27 kDa.

14. Composition selon la revendication 11, dans laquelle l'agent est sélectionné de manière à résulter après clivage en ce qu'au moins 90 % des fragments de protéine après clivage ont un poids moléculaire inférieur à 25 kDa.

15. Composition selon la revendication 11, dans laquelle l'agent est sélectionné de manière à résulter après clivage en ce qu'au moins 90 % des fragments de protéine après clivage ont un poids moléculaire inférieur à 23 kDa.
